# EUROPEAN PATENT APPLICATION

(11) **EP 1 739 180 A1**
(43) Date of publication of application: **03.01.2007**
(21) Application number: 05013833.8
(22) Date of filing: 27.06.2005
(51) Int. Cl.: C12N 15/867, C12N 5/06, C12N 5/10

(54) **T cell line-based packaging cell line for the production of retroviruses by enriching of CD3 expressing cells**

(71) Applicant: Max-Delbrück-Centrum für Molekulare Medizin (MDC), 13092 Berlin (DE)
(72) Inventor: Uckert, Wolfgang, 13125 Berlin (DE); Blankenstein, Thomas, 13467 Berlin (DE)
(74) Representative: Krauss, Jan

(57) **Abstract**

The present invention relates to retroviral vector systems, in particular the development of packaging or helper cell lines that are based on T cells and that are suitable for producing T cell receptor retroviruses. Thereby, the present invention relates to T cell line specific retroviral expression vectors, and mammalian T cell line-based helper cell lines. The invention further relates to methods for expressing transgenes and to T cell line specific retroviral vector systems, including biochemical kits, that utilize and/or comprise the T cell line specific retroviral expression vectors and/or mammalian T cell line-based helper cell lines.

## Description

The present invention relates to retroviral vector systems, in particular the development of packaging or helper cell lines that are based on T cells and that are suitable for producing T cell receptor retroviruses. Thereby, the present invention relates to T cell line specific retroviral expression vectors, and mammalian T cell line-based helper cell lines. The invention further relates to methods for expressing transgenes and to T cell line specific retroviral vector systems, including biochemical kits, that utilize and/or comprise the T cell line specific retroviral expression vectors and/or mammalian T cell line-based helper cell lines.

### Description

The concept of gene therapy involves the transfer of genetic material into a cell, tissue, or whole organ, with the goal of curing a disease or at least improving the clinical status of a patient. A key factor in the success of gene therapy is the development of delivery systems that are capable of efficient gene transfer in a variety of tissues, without causing any associated pathogenic effects. Vectors based upon many different viral systems, including retroviruses, lentiviruses, adenoviruses, and adeno-associated viruses, currently offer the best choice for efficient gene delivery.

### Retroviral gene transfer technology

Retroviral gene transfer is a technique for efficiently introducing stable, heritable genetic material into the genome of any dividing cell type. Retroviruses are an efficient meals to deliver single DNA expression constructs to a wide range of mammalian cell types. They are by far the easiest and fastest means to deliver genes stably to mammalian cells.

Current retroviral gene transfer technology is based on the coordinated design of packaging cell lines and retroviral expression vectors. The development of packaging cell lines, also called helper cell lines, which are cells that package recombinant retroviral RNAs into infectious, replication-incompetent particles, created a new level of safety and control (Mann R, Mulligan RC, & Baltimore D. Construction of retrovirus packaging mutant and its use to produce helper-free defective retrovirus. Cell 1983, 33:153-9., Miller AD, & Buttimore C. Redesign of retrovirus packaging cell lines to avoid recombination leading to helper virus production. Mol Cell Biol. 1986, 6(8):2895-902.).

### Packaging cells

To develop a packaging cell line, the viral *gag, pol* and *env* genes, which are necessary for particle formation and replication, are stably integrated into the genome of the packaging cell line. However, one of the requirements for the use of retroviral vectors in e.g. human gene therapy is a packaging cell line which is incapable of producing replication-competent virus but still produces high titers of replication-deficient vector virus. Therefore, the structural genes are separately introduced and integrated (e.g. viral *gag* and *pol* genes are on one plasmid and the viral *env* gene is on another plasmid) for minimizing the chances of producing replication-competent virus due to recombination events during cell proliferation (Morgenstern JP, & Land H. Advanced mammalian gene transfer: high titre retroviral vectors with multiple drug selection markers and a complementary helper-free packaging cell line. Nucleic Acids Res. 1990, 18(12):3587-96., Miller AD, & Chen F. Retrovirus packaging cells based on 10A1 murine leukemia virus for production of vectors that use multiple receptors for cell entry. J. Virol. 1996, 70(8):5564-71.).

The viral *env* gene, expressed by the packaging cell line, encodes the envelope protein, which determines the range of infectivity (tropism) of the packaged virus. Viral envelopes are classified according to the receptors used to enter the host cells. For example, ecotropic virus can recognize a receptor found on only mouse and rat cells. Amphotropic virus recognizes a receptor found on a broad range of mammalian cell types. Dualtropic virus recognizes two different receptors found on a broad range of mammalian cell types.

Most commonly adherent cell lines have been used for the generation of packaging cells. Recently, the development of packaging cells that grow in suspension has been described, because the large-scale production of high titre retrovirus vectors could benefit from the development of packaging cells growing in suspension. In these studies also T cells have been used as packaging cell lines. So describe Chan *et al.* the development of a stable suspension packaging cell line derived from human lymphoblastoid cells WIL-2 for the production of high-titre retroviral vectors (Chan LM, Coutelle C, & Themis M. A novel human suspension culture packaging cell line for production of high-titre retroviral vectors. Gene Ther. 2001, 8(9):697-703.) Furthermore, Pizzato *et al.* developed T as well as B lymphoidblastoid cell lines as packaging cell lines that grow in suspension (Pizzato M, Merten OW, Blair ED, & Takeuchi Y. Development of a suspension packaging cell line for production of high titre, serum-resistant murine leukemia virus vectors. Gene Ther. 2001, 8(10):737-45.).

T cell lines express the CD3 complex and can transport T cell receptor (TCR) chains to the cell surface. The TCR/CD3 complex is then formed on the cell surface. The TCR/CD3 complex is a multimeric protein complex composed of a minimum of seven transmembrane chains, which are TCR alpha (TCR α), TCR beta (TCR β), CD3 gamma (CD3 γ), CDR delta (CD3 δ), CD3 epsilon (CD3 ε), and CD3 zeta 2 (CD3 ζ). Earlier studies have demonstrated that both the TCR alpha and TCR beta chains are required for the cell surface expression of the TCR/CD3 complex. For a role of the CD3 chains for the TCR/CD3 expression in human T cells CD3 zeta is required for the cell surface expression of the TCR/CD3 complex and CD3 zeta is required for the export of the TCR/CD3 complex from the endoplasmic reticulum to the Golgi apparatus for subsequent processing (Geisler C, Kuhlmann J, & Rubin B.: Assembly, intracellular processing, and expression at the cell surface of the human alpha beta T cell receptor/CD3 complex. Function of the CD3-zeta chain. J. Immunol. 1989, 143(12):4069-77.).

In packaging cells that are based on T cell lines, cells producing TCR vector particles can be identified and enriched by means of mAbs. One requirement for this method is, nevertheless the availability of antibodies that are specific for the respective TCR α or TCR β chain. However, such antibodies are not available for all TCR chains.

Therefore, the objective of the present invention is the development of improved methods for the generation of packaging T cell lines that allow for a simple and improved identification of packaging T cells producing vector particles, such as packaging T cell lines producing a high-titre of TCR vector particles.

This problem is solved by the present invention in a first aspect thereof by providing a T cell line specific retroviral expression vector.

The T cell line specific retroviral expression vector according to the present invention comprises the packaging signal for the mobilisation of vector particles. The T cell line specific retroviral expression vector according to the present invention further comprises a CD3 complex deficiency complementing gene or transgene that is to be transferred and expressed. The T cell line specific retroviral expression vector according to the present invention further optionally comprises a second transgene that is to be transferred and expressed. The T cell line specific retroviral expression vector according to the present invention also comprises cis-elements for expressing of said CD3 complex deficiency complementing gene or transgene and of said second transgene.

Packaging signals and cis-elements suitable for retroviral vectors are known to the person of skill in the art. Packaging signals are preferably derived or used from the respective retroviruses originally used for designing the vector. Preferred *cis*-elements are among others viral long term repeat (LTR) structures, locus control region (LCR) elements and posttranscriptional regulatory elements (PRE), such as woodchuck hepatitis virus PRE.

Exemplary packaging signals and *cis*-elements are e.g. disclosed in Engels B, Cam H, Schuler T, Indraccolo S, Gladow M, Baum C, Blankenstein T, & Uckert W. Retroviral vectors for high-level transgene expression in T lymphocytes. Human Gene Ther. 2003, 14:1155-68; Engels B, Noessner E, Frankenberger B, Blankenstein T, Schendel DJ, & Uckert W. Redirecting human T lymphocytes toward renal cell carcinoma specificity by retroviral transfer of T cell receptor genes. Human Gene Ther. 2005, 16:790-810; Verma IM, & Weitzman MD. GENE THERAPY: Twenty-First Century Medicine. Annual Review of Biochemistry. 2005. 74: 711-738; Kootstra NA, & Verma IM. GENE THERAPY WITH VIRAL VECTORS. Annual Review of Pharmacology and Toxicology. 2003, 43: 413-439; Hu WS, & Pathak VK. Design of retroviral vectors and helper cells for gene therapy. Pharmacol Rev. 2000, 52(4):493-511, and Robbins PD, & Ghivizzani SC. Viral vectors for gene therapy. Pharmacol Ther. 1998, 80(1):35-47.

The CD3 complex deficiency complementing gene that is to be transferred and expressed is also preferably a foreign gene or transgene. It is further preferred that the CD3 complex deficiency complementing gene or transgene of the T cell line specific retroviral expression vector is selected from the group of at least one of a TCR α chain, a TCR β chain, a TCR α chain and a TCR β chain, CD3 gamma (CD3 γ), CDR delta (CD3 δ), CD3 epsilon (CD3 ε), and CD3 zeta (CD3 ζ). Suitable preferred TCR chains are specific to tumors or viruses.

The retroviral vector is designed to be suitable for any kind of possible transgene. Preferred (optional) second transgenes of the retroviral vector according to the invention are GFP and similar marker genes, such as Luciferase. Preferred second transgenes are also therapeutic genes, such as TCR α chain, TCR β chain, TCR α chain and TCR β chain, or any other therapeutic gene.

Furthermore, the T cell line specific retroviral expression vector according to the invention preferably comprises an additional selection marker. Preferred selection markers are antibiotic resistance markers, such as Neomycin, Puromycin, Hygromycin or Zeocin. Suitable selection markers are known to the skilled person.

The introduction of an additional selection gene into the retroviral vector can be used in order to identify, isolate and expand those packaging cells that were successfully transfected and produce vector particles. In applications where the expression of a selection gene is undesired, vector particle-producing packaging cells could also be identified by the expression of the product of a transgene or foreign gene. A requirement therefore is either the possibility of a direct detection of the product of the transgene (e.g. with green fluorescent protein, GFP) or an indirect detection using monoclonal antibodies (mAbs) that bind to the product of the transgene. Since the expression level of the products of the transgene in the packaging cells directly correlates with the production level of vector particles, in this way packaging cell lines having a high production of vector particles can be isolated. The use of mAbs can also advantageously be combined with cell sorting techniques for the detection and enrichment of transgene expressing packaging cells. However, such a combination can only be employed when the protein that is encoded by the transgene is transported to the surface of the packaging cell.

The invention further provides a mammalian T cell helper cell line. The T cell helper cell line is deficient in at least one gene that is required for a presentation of the CD3 complex on the cell surface. Thereby, the marker is preferably selected from the group of the genes for a TCR α chain, a TCR β chain, a TCR α chain and a TCR β chain, CD3 γ, CD3 δ, CD3 ε, and CD3 ζ.

Previously established packaging or helper cell lines that are based on T cell lines are not deficient in the TCR α chain, the TCR β chain or both the TCR α chain and the TCR β chain. Therefore, these cells already express CD3 molecules on their cell surface without being transfected by retrovirus plasmids coding for TCR. After transferring TCR retrovirus plasmids into these packaging cells "false" positive packaging cells are detected by an anti-CD3 mAb, i.e. also such cells that do not produce vector particles. Therefore, in these previously established packaging cell lines the expression of CD3 molecules cannot be used for the identification and enrichment of packaging cells producing TCR vector particles.

It is further preferred, that the mammalian T cell helper cell line is a *gag-pol* gene positive and *env* gene positive Δα Jurkat cell, Δβ Jurkat cell or Δα/Δβ Jurkat cell. Preferred mammalian T cell helper cell lines are of human or mouse origin. Suitable mouse cell lines are 58 or TG40. In general, cell lines that are CD3 negative are suitable.

Preferred *gag* and *pol* gene sequences are derived from Moloney mouse leukaemia virus (Mo-MLV). Preferred *env* gene sequences are derived from gibbon ape leukaemia virus (GALV), ecotropic and amphotropic mouse leukaemia viruses, such as 10A1 (MLV-10A1), feline endogenous virus RD114, vesicular stomatitis virus.

The mammalian T cell helper cell line according to the invention preferably expresses a transfected CD3 complex deficiency complementing gene or transgene and optionally expresses a transfected transgene. Preferred CD3 complex deficiency complementing genes and transgenes are discussed above.

The invention further provides a non-human transgenic animal, comprising a T cell helper cell line according to the invention.

The invention further provides a method for expressing a CD3 complex deficiency complementing gene or transgene and optionally a second transgene. The method comprises the steps of:
a) providing a T cell line specific retroviral expression vector according to the invention comprising the CD3 complex deficiency complementing gene or transgene to be expressed and optionally the second transgene to be expressed,
b) providing a mammalian T cell helper cell line according to the invention,
c) transfecting said helper cell line with said retroviral expression vector, and
d) selecting helper cells expressing said CD3 complex deficiency complementing gene or transgene and optionally said second transgene based on the presentation of the CD3 complex on the surface of said cell.

It is preferred, that the selecting of step d) of the method according to the invention comprises a TCR chain specific and/or a CD3 specific antibody.

It is furthermore preferred that the selecting of step d) comprises selecting by means of an immunoaffinity method. Suitable immunoaffinity methods are known to a person skilled in the art. Examples are FACS or MACS.

In a preferred embodiment, the method further comprises a purification of the expression product of the CD3 complex deficiency complementing gene or transgene and optionally of the expression product of the second transgene from said selected cell and/or the vector particles as produced by said selected cell.

The invention further provides a T cell line specific retroviral vector system, comprising a retroviral vector according to the invention and a T cell helper cell line according to the invention.

The invention further provides a biochemical kit for a CD3 complex deficiency complementing gene or transgene and optionally expressing a second transgene, comprising a T cell line specific retroviral vector system according to the invention.

Retroviral vector systems consist of two components: The first component, the *retroviral vector,* is derived from a retrovirus and should not code for viral genes. The vector genome carries the packaging signal that is important for the mobilization of vector particles, at least one foreign gene (transgene) to be transferred, and the regulatory sequences (*cis*-elements) that are required for the expression of the foreign gene. The second component, the *packaging cell line* (helper cell line), carries the helper viral genome, that is also derived from a retrovirus. It encodes only for those proteins that are required for the assembly, the maturation, and the gene transferring functions of the virus (*trans*-elements). The helper virus genome is constructed in that the recognition sequences (packaging signals) that are required for the mobilization as well as the cis-regulatory sequences for the reverse transcription and integration are removed in the viral particle. Packaging cells are produced by a step-wise transfer of the different components of the helper genome (such as *gag-pol* gene, *env* gene) into, most commonly, adherently growing cells of human and mouse origin.

If a retroviral vector in its common form as plasmid-DNA is transfected into packaging cells, the packaging cells produce vector particles. The vector particles carry the genome of the foreign gene(s) and are infectious, i.e. they can infect cells and thus transfer the transgene(s) into these cells. No replication of the virus occurs in the infected cells and therefore no release of further vector particles. In parallel to the production of vector particles in the packaging cell the genetic product of the transgene(s) is also synthesized, since the genome of the vector is present as messenger RNA (mRNA) in the cells.

The inventors have constructed retroviral vectors that encode for the α and β chain of T cell receptors (TCR) as foreign gene and do not contain a selection gene. The transfer of the vector genome into packaging cells leads to a production of vector particles. A simple selection of packaging cells producing TCR vector particles by using mAbs is not possible when adherently growing packaging cells are used due to the fact that these cells do not have the components that are required for the surface expression of TCR chains. Such components are the components of the CD3 complex, γ, δ, ε, ζ chains.

Therefore, the inventors have started from T cell lines that either exhibit a deficiency in the α chain (e.g. Δα Jurkat) or β chain (e.g. Δβ Jurkat) of the CD3 complex and that preferably grow in suspension. These cells can synthesize all components of the CD3 complex, but cannot transport the CD3 complex to the cell surface due to the missing α or Δβ chain, respectively, and thus are unable to react with anti-CD3 mAbs. After transfer of a TCR retroviral plasmid into these cells, that are designated as CD3 negative, TCR α and TCR β chain proteins are synthesized in the cells, and now all components for the surface expression of the CD3 complex are available. Thus, CD3 positive cells that can be identified with anti-CD3 mAbs are derived from CD3 negative cells. Using subsequent FACS sorting, those cells can be enriched that show a particular strong expression of the CD3 complex and that produce large amounts of vector particles with a high probability.

The methods and packaging cell lines of the invention allow for an identification of packaging cells producing vector particles
- that is independent on the TCR chain specificity
- by means of the expression of the CD3 complex and anti-CD3 mAbs.

Following this strategy, packaging cells producing a high titre of vector particles can be generated without being required to perform a labour, time, and cost intensive cloning of packaging cells and subsequent characterization of a large number of cell clones.

The following Figures and Examples are included for further illustrating and exemplifying the invention.

### Figures

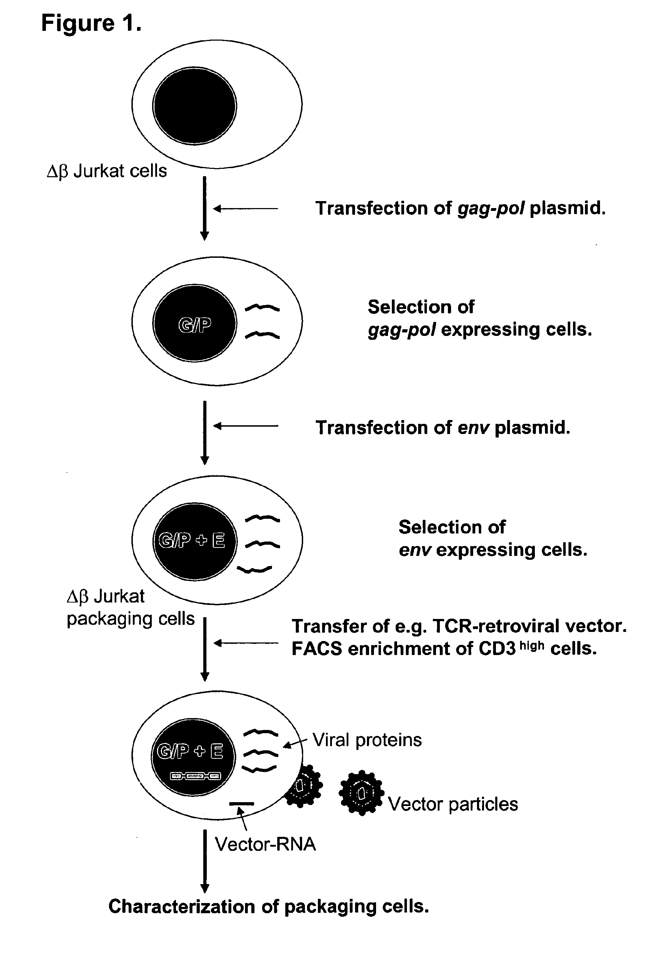

**Figure 1** illustrates the construction of a T cell line-based packaging cell line according to the invention, in a flow chart.
   The generation of *gag-pol* positive and *env* positive Δβ Jurkat packaging cell lines is shown.
   After the transfection of Δβ Jurkat cells with a *gag-pol* plasmid (e.g. Mo-MLV) *gag-pol* expressing cells are selected, cell cloning by limited dilution and analysis of cell clones for *gag* gene coded protein expression and RT activity are carried out. Then, these selected cells are transfected with an *env* plasmid (e.g. GALV) followed by selection of *env* expressing cells, cell cloning by limited dilution and analysis of cell clones for *env* gene coded protein expression. In the next step the retroviral vector is transferred, e.g. TCR-retroviral vector. Then, FACS enrichment of CD3 high cells is carried out. Packaging cells are then characterized by e.g. vector titre, replication competent retrovirus.

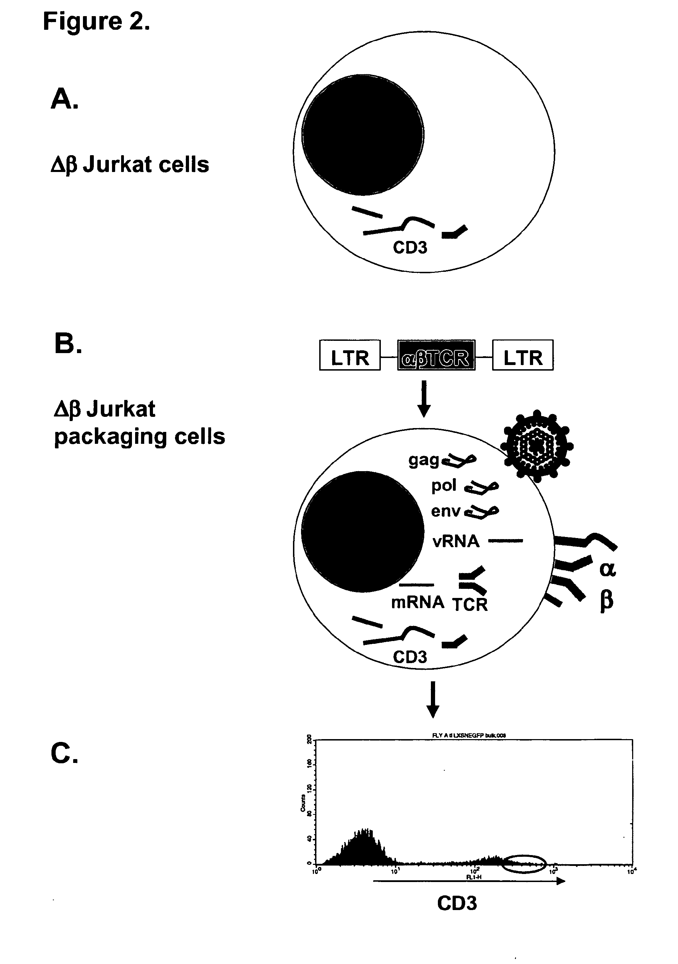

**Figure 2** shows the application of "de novo" CD3 expression for the generation of high vector titre producing T cell line-based packaging cells according to the invention.
   **A.** Δβ Jurkat cells are deficient in CD3 cell surface expression.
   **B.** In Δβ Jurkat packaging cells (e.g. Δβ Jurkat/GALV) CD3 cell surface expression takes place after transfer of a TCR gene encoding retroviral vector.
   **C**. CD3 high expressing packaging cells are FACS enriched, expanded, and used as vector particle producing cells.

### Examples

### Example 1 Production of the vector

The production of the vector can be carried out as disclosed in Engels *et al.* 2003 (Engels B, Cam H, Schuler T, Indraccolo S, Gladow M, Baum C, Blankenstein T, & Uckert W. Retroviral vectors for high-level transgene expression in T lymphocytes. Human Gene Ther. 2003, 14:1155-68.) or in Engels *et al.* 2005 (Engels B, Noessner E, Frankenberger B, Blankenstein T, Schendel DJ, & Uckert W. Redirecting human T lymphocytes toward renal cell carcinoma specificity by retroviral transfer of T cell receptor genes. Human Gene Ther. 2005, 16:790-810.)

### Example 2 Production of the packaging cells

Exemplary, the inventors transferred the *gag-pol* sequences of the Moloney mouse leukaemia virus (Mo-MLV) by electroporation of the plasmid vector pGP into Δβ Jurkat cells. The vector contains the selection gene Blasticidine 5 and allows for the selection of positively transfected cells. Subsequently the surviving cells are cloned by "limited" dilution in order to obtain individual cell clones. The separate cell clones are expanded and the expression of the *gag* and *pol* gene products is analysed on the transcription level by RT PCR and on the translation level by Western blot analysis. For an analysis of the expression level of the *gag* gene products by RT PCR, RNA is isolated from the transfected cells and translated into cDNA. Then a PCR with *gag* specific ologonucleotide primers is carried out. For an analysis of the expression level of the *gag* gene products by Western blot cell lysates are produced. The proteins are separated by polyacrylamide gel electrophoresis and transferred onto a carrier that is incubated with mAbs recognizing protein p30 that is encoded by the *gag* gene. The detection of the expression of the *pol* gene product reverse transcriptase is carried out by means of an assay wherein the enzymatic activity of this protein is measured (revertase assay).

The *env* gene of the gibbon ape leukaemia virus (pALV-GALV) or the amphotropic mouse leukaemia virus 10A1 (pALF-10A1), respectively, was transfected by electroporation into the Δβ Jurkat cell clones that exhibited the highest *gag-pol-*gene expression. (Retroviruses with the envelope of GALV or MLV-10A1, respectively, particularly effectively infect different immune cells, amongst others primary human T-lymphocytes, haematopoietic stem cells, and dendritic cells.) These plasmid vectors include the selection marker Phleomycin and also allow for a selection of positively transfected cells. After cloning of the cells (see above) the expression of the *env* gene product gp70 is determined by RT PCR and Western blot analysis using mAbs that are directed against gp70. Cell clones having the highest *env* expression were expanded and constituted the novel packaging cell line.

According to the same method the *env* genes of other retroviruses can be transferred into *gag-pol* gene-positive Δβ Jurkat cell clones, in order to generate retroviral vector particles with different viral envelopes (pseudotypes).

### Example 3 Production of vector particles

The viral production by the different packaging cells was first analysed with a retroviral vector containing GFP as foreign gene. The retroviral vector MP71 GFPPRE was transfected into the individual packaging cells by electroporation. After 72 to 96 hours the packaging cells were enriched by FACS sorting, whereby the high fluorescing cellular fraction was isolated and expanded. These cells were analysed for the production of retroviral vector particles.

In additional experiments the retroviral vector carrying the α and β chain of a TCR, such as MP71 TCR26 as disclosed in Engels *et al.* 2005 (supra), is transferred by electroporation into the different packaging cells. After 72 to 96 hours the packaging cells are labelled with anti-CD3 mAb. Cells expressing CD3 were enriched by FACS sorting, expanded and analysed for production of vector particles. Since an mAb (anti-Vβ22) being directed against the β chain of the TCR26 is available, the inventors examined, whether the production of vector particles CD3 enriched packaging cells correlated with the Vβ22 enriched cells.

The TCR26 vector particles produced by the novel packaging cell lines were used for the transduction of primary human T lymphocytes. This allowed for an analysis of the expression of TCR26 in these lymphocytes.

The inventors analysed the packaging cell line for the formation of replication competent retroviruses (PCR, analysis of the cell culture supernatant) and developed methods for the generation of cell culture supernatants with high viral titres.

## Claims

1. A T cell line specific retroviral expression vector, comprising
a) the packaging signal for the mobilisation of vector particles,
b) a CD3 complex deficiency complementing transgene to be transferred and expressed,
c) optionally, a second transgene to be transferred and expressed.
c) *cis*-elements for expressing of said CD3 complex deficiency complementing transgene and of said second transgene.

2. The T cell line specific retroviral expression vector according to claim 1, furthermore comprising an additional selection marker.

3. The T cell line specific retroviral expression vector according to claim 1 or 2, wherein said CD3 complex deficiency complementing transgene is selected from the group of at least one of a TCR α chain, a TCR β chain, a TCR α chain and a TCR β chain, CD3 γ, CD3 δ, CD3 ε, and CD3 ζ.

4. A mammalian T cell helper cell line, wherein said T cell helper cell line is deficient in at least one gene that is required for a presentation of the CD3 complex on the cellular surface.

5. The mammalian T cell helper cell line according to claim 4, wherein said gene is selected from the group of the genes for a TCR α chain, a TCR β chain, a TCR α chain and a TCR β chain, CD3 γ, CD3 δ, CD3 ε, and CD3 ζ.

6. The mammalian T cell helper cell line according to claim 4 or 5, wherein said cell line is a *gag-pol* gene positive and *env* gene positive Δα Jurkat cell, Δβ Jurkat cell or Δα/Δβ Jurkat cell.

7. The mammalian T cell helper cell line according to any of claims 4 to 6 expressing a transfected CD3 complex deficiency complementing transgene and optionally a transfected second transgene according to claim 1.

8. A transgenic non-human animal, comprising a T cell helper cell line according to any of claims 4 to 7.

9. A method for expressing a CD3 complex deficiency complementing transgene and optionally a second transgene, comprising the steps of:
a) providing a T cell line specific retroviral expression vector according to any of claims 1 to 3 comprising the CD3 complex deficiency complementing transgene to be expressed and optionally the second transgene to be expressed,
b) providing a mammalian T cell helper cell line according to any of claims 4 to 6,
c) transfecting said helper cell line with said retroviral expression vector, and
d) selecting helper cells expressing said CD3 complex deficiency complementing transgene and optionally said second transgene based on the presentation of the CD3 complex on the surface of said cell.

10. The method according to claim 9, wherein said selecting comprises a TCR chain and/or a CD3 specific antibody.

11. The method according to claim 9 or 10, wherein said selecting comprises selecting by means of an immuno-affinity method, such as FACS or MACS.

12. The method according to any of claim 9 to 11, further comprising a purification of the expression product of the CD3 complex deficiency complementing transgene and optionally the second transgene from said selected cell and/or the vector particles as produced by said selected cell.

13. A T cell line specific retroviral vector system, comprising a retroviral vector according to any of claims 1 to 3 and a T cell helper cell line according to any of claims 4 to 6.

14. A biochemical kit for expressing a CD3 complex deficiency complementing transgene and optionally a second transgene, comprising a T cell line specific retroviral vector system according to claim 13.
